(19) Europäisches Patentamt
European Patent Office
Office européen des brevets

(11) Numéro de publication : **0 319 406 B1**

(12) **FASCICULE DE BREVET EUROPEEN**

(45) Date de publication du fascicule du brevet : **18.03.92 Bulletin 92/12**

(51) Int. Cl.$^5$ : **C07C 29/136,** C07C 29/14, C07C 33/02

(21) Numéro de dépôt : **88403016.4**

(22) Date de dépôt : **30.11.88**

(54) **Procédé de préparation d'alcools insaturés.**

(30) Priorité : **01.12.87 FR 8716628**

(43) Date de publication de la demande : **07.06.89 Bulletin 89/23**

(45) Mention de la délivrance du brevet : **18.03.92 Bulletin 92/12**

(84) Etats contractants désignés : **AT BE CH DE ES FR GB GR IT LI LU NL SE**

(56) Documents cités :
**EP-A- 0 024 648**
**EP-A- 0 061 337**
**GB-A- 2 079 278**
**US-A- 3 968 147**

(73) Titulaire : **RHONE-POULENC SANTE**
**20, avenue Raymond Aron**
**F-92160 Antony (FR)**

(72) Inventeur : **Grosselin, Jean-Michel**
**15 rue Terraille**
**F-69001 Lyon (FR)**
Inventeur : **Mercier, Claude**
**85, avenue du Point du Jour**
**F-69005 Lyon (FR)**

(74) Mandataire : **Le Pennec, Magali et al**
**RHONE-POULENC SANTE, Service Brevets,**
**20 Avenue Raymond Aron**
**F-92165 Antony Cédex (FR)**

**EP 0 319 406 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de préparation d'alcools insaturés primaires ou secondaires par hydrogénation des aldéhydes ou des cétones correspondants.

En effet, l'objet de la présente invention concerne un procédé pour la préparation des alcools insaturés de formule générale :

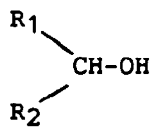

(I)

dans laquelle $R_1$ et $R_2$, identiques ou différents, représentent un atome d'hydrogène ou un radical aliphatique saturé ou non saturé, éventuellement substitué par un radical alicyclique saturé ou non saturé ou par un radical aromatique, ou un radical alicyclique non saturé ou un radical aromatique, ou bien $R_1$ et $R_2$ forment ensemble un radical alicyclique saturé ou non saturé, étant entendu que :

- $R_1$ et $R_2$ ne peuvent pas représenter simultanément un atome d'hydrogène,
- l'un au moins des radicaux $R_1$ ou $R_2$ contient une double liaison éthylénique,
- les radicaux aliphatiques, alicycliques ou aromatiques peuvent être éventuellement substitués par un ou plusieurs radicaux, identiques ou différents, choisis parmi les radicaux alcoyles contenant 1 à 4 atomes de carbone, hydroxy ou alcoxy dont la partie alcoyle contient 1 à 4 atomes de carbone, par hydrogénation sélective au moyen de l'hydrogène d'un composé carbonylé de formule générale :

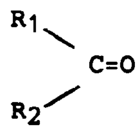

(II)

dans laquelle $R_1$ et $R_2$ sont définis comme ci-dessus, caractérisé en ce que l'on opère en milieu liquide homogène en présence d'un catalyseur à base de ruthénium choisi parmi les hydrures et les dérivés halogénés du ruthénium complexés par un ligand ou associés à un ligand éventuellement générés in situ.

Plus particulièrement, la présente invention concerne la préparation d'alcools $\alpha,\beta$-insaturés à partir des composés carbonylés $\alpha,\beta$-insaturés correspondants, c'est-à-dire la préparation des produits de formule générale (I) dans laquelle un au moins des symboles $R_1$ ou $R_2$ contient une double liaison en position $\alpha,\beta$ par rapport à la fonction alcool à partir des composés $\alpha,\beta$-insaturés de formule générale (II) correspondants.

Plus particulièrement, la présente invention concerne la préparation des alcools $\alpha,\beta$-insaturés de formule générale (I) dans laquelle un des symboles $R_1$ ou $R_2$ représente un atome d'hydrogène et l'autre représente un radical aliphatique contenant 1 à 30 atomes de carbone et au moins une double liaison en position $\alpha,\beta$ par rapport à la fonction alcool éventuellement substitué par un ou plusieurs radicaux, identiques ou différents, choisis parmi les radicaux alcoyles contenant 1 à 4 atomes de carbone, hydroxy ou alcoxy dont la partie alcoyle contient 1 à 4 atomes de carbone, ou par un radical alicyclique contenant 5 ou 6 atomes de carbone saturé ou non saturé éventuellement substitué par un ou plusieurs radicaux alcoyles contenant 1 à 4 atomes de carbone, ou par un radical phényle éventuellement substitué, ou bien $R_1$ et $R_2$ forment ensemble un radical alicyclique non saturé éventuellement substitué par un ou plusieurs radicaux alcoyles contenant 1 à 4 atomes de carbone, à partir des composés carbonylés $\alpha,\beta$-insaturés de formule générale (II) correspondants.

Plus particulièrement, la présente invention concerne la préparation du prénol à partir du prénal, du nérol/géraniol à partir du citral, de l'alcool crotylique à partir du crotonaldéhyde ou de l'alcool cinnamique à partir du cinnamaldéhyde.

Alors que l'hydrogénation d'une double liaison carbone-carbone est facilement réalisable en catalyse homogène, la réduction de la fonction carbonyle s'effectue difficilement surtout dans le cas des dérivés carbonylés insaturés.

Il est connu d'effectuer la réduction d'aldéhydes saturés en présence d'un métal du groupe du platine, d'un ligand hydrosoluble et d'un composé amphiphile (EP 61337), ou d'un dérivé carboxylé du ruthénium (GB 2 079 278). Le brevet européen EP 24648 décrit l'hydrogénation en phase liquide d'aldéhydes insaturés en présence d'un métal supporté et d'une amine tertiaire. Le brevet US 3 968 147 décrit la préparation d'alcools optiquement actifs par hydrogénation de cétones optiquement inactives au moyen d'un catalyseur comprenant un métal du

groupe VIII complexé à une phosphine optiquement active.

Il est aussi connu d'utiliser des complexes organo-métalliques à base de rhodium [T. Mizoroki et coll., Bull. Chem. Soc. Japan, 50, 2148 (1977)] ou d'iridium [E. Farnetti et coll., J. Chem. Soc. Chem. Comm., p. 746 (1986)] pour réduire sélectivement l'aldéhyde cinnamique en alcool insaturé correspondant. W. Strohmeier et K. Kolke, J. Organometal. Chem., 193, C63 (1980) ont décrit la réduction de l'aldéhyde crotonique au moyen de complexes du ruthénium, la meilleure sélectivité étant obtenue avec $RuCl_2$ $[P(C_6H_{11})_3]_2$ $(CO)_2$.

Enfin K. Hotta, J. Mol. Catal., 29, 105-107 (1985) a montré que le citral était transformé en géraniol/nérol par hydrogénation en présence d'un complexe $RuCl_2$ $[(PPh_3)]_3$ en opérant dans un mélange toluène-éthanol en présence d'un excès d'acide chlorhydrique.

Il a maintenant été trouvé, et c'est ce qui fait l'objet de la présente invention, que les composés carbonylés de formule générale (II) peuvent être transformés sélectivement en alcools de formule générale (I) par hydrogénation, en milieu homogène, en présence d'un catalyseur choisi parmi les hydrures et les dérivés halogénés du ruthénium complexés par un ligand ou associés à un ligand éventuellement générés in situ.

Parmi les dérivés du ruthénium ceux qui conviennent particulièrement bien sont $RuH_2$ $(PPh_3)_4$ qui peut être préparé selon R.O. Harris et coll., J. Organometal. Chem., 54, 259-264 (1973) ou RuH OAc $(PPh_3)_4$ qui peut être préparé selon Inorg. Synthesis, 16, p. 53 et 75 à 79 ou $RuCl_2$ $(PPh_3)_4$.

Des résultats intéressants sont également obtenus en utilisant des dérivés du ruthénium choisi parmi les sels minéraux ou les oxydes de ruthénium en présence d'un ligand choisi, de préférence, parmi les phosphines telles que la triphénylphosphine ($PPh_3$) ou la tri(métasulfophényl) phosphine (TPPTS). Peut avantageusement être utilisé un système catalytique résultant de l'association d'un chlorure de ruthénium ($RuCl_3$, $xH_2O$) et de la triphénylphosphine ou de la tri(métasulfophényl) phosphine.

Généralement l'hydrogénation est réalisée à une température comprise entre 0 et 100°C, de préférence entre 20 et 50°C, en opérant dans un solvant organique. Il est particulièrement avantageux d'opérer sous une pression comprise entre 1 et 200 bars, de préférence entre 1 et 20 bars.

Comme solvant organique, on utilise de préférence un solvant polaire choisi parmi les alcools (méthanol, éthanol, isopropanol) éventuellement associé à un solvant non polaire choisi parmi les hydrocarbures aliphatiques (pentane, hexane, heptane, octane), alicycliques (cyclohexane), aromatiques (benzène, toluène, xylène), les éthers (éther diéthylique, diisopropylique) et les esters (acétate de méthyle, acétate d'éthyle, acétate de butyle).

Il est particulièrement avantageux d'ajouter au solvant polaire ou au mélange de solvants une quantité d'eau qui peut atteindre jusqu'à 35 % du volume total de telle manière que le milieu reste homogène.

Généralement, on utilise de 0,1 à 0,001 mole de catalyseur par mole d'aldéhyde $\alpha,\beta$-insaturé de formule générale (II).

Le procédé selon la présente invention permet d'obtenir les alcools insaturés de formule générale (I) avec une sélectivité qui est généralement supérieure à 80 %.

Les exemples suivants, donnés à titre non limitatif, montrent comment l'invention peut être mise en pratique.

## EXEMPLE 1

Dans un réacteur de type SOTELEM de 300 cm3 préalablement purgé à l'azote, on introduit successivement 0,50 g de $RuH_2$ $(PPh_3)_4$ (0,43 mmole), 8,7 g de prénal (0,103 mole), 82 g d'isopropanol et 4 g d'eau. On purge 3 fois à l'azote puis 3 fois à l'hydrogène sous une pression de 20 bars. On fixe alors la pression d'hydrogène à 30 bars puis chauffe le mélange réactionnel à 50°C. On règle l'agitation à 1200 tours/minute.

Après 2 heures 40 minutes, l'analyse par chromatographie en phase gazeuse (colonne Rheoplex 400 ; Chromosorb PAW ; longueur 2,5 m ; diamètre 3 mm ; analyse isotherme à 130°C) montre que :

– le taux de transformation du prénal est de 100 %

– la répartition des produits d'hydrogénation est la suivante :

| | |
|---|---|
| . prénol | : 90,6 % |
| . alcool isoamylique | : 4 % |
| . isovaléraldéhyde | : non détectable |

## EXEMPLE 2

On opère comme dans l'exemple 1 mais en utilisant 0,43 mmole de $RuCl_2$ $(PPh_3)_4$.

Après 180 minutes, l'analyse par chromatographie en phase gazeuse montre que :

– le taux de transformation du prénal est de 80 %

– le répartition des produits d'hydrogénation est la suivante :

. prénol : 65 %
. alcool isoamylique : 7 %
. isovaléraldéhyde : 4 %

La sélectivité en prénol est de 81 %.

EXEMPLE 3

On opère comme dans l'exemple 1 mais en utilisant 0,36 mmole de RuH OAc $(PPh_3)_3$.

Après 7 heures 10 minutes, l'analyse par chromatographie en phase gazeuse montre que :

– le taux de transformation du prénal est de 80 %

– la répartition des produits d'hydrogénation est la suivante :

. prénol : 44 %
. alcool isoamylique : 5 %
. isovaléraldéhyde : 4 %

La sélectivité en prénol est de 73 %.

EXEMPLE 4

Dans une ampoule de verre de 25 cm3, on introduit successivement :

- $RuCl_3$, $3H_2O$ 9,5 x $10^{-5}$ mole
- triphénylphosphine 4,24 x $10^{-4}$ mole
- isopropanol 8,5 g
- eau 1 g
- prénal 19 x $10^{-3}$ mole

L'ampoule est introduite dans un autoclave de 125 cm3 placé dans une enceinte permettant une agitation par secousses. On purge 3 fois avec de l'hydrogène puis on fixe la température à 35°C et la pression à 23 bars.

Après 2 heures 40 minutes, l'analyse par chromatographie en phase gazeuse montre que :

– le taux de transformation du prénal est de 72 %

– la répartition des produits d'hydrogénation est la suivante :

. prénol : 70 %
. alcool isoamylique : 1,2 %
. isovaléraldéhyde : 0,6 %

La sélectivité en prénol est de 97 %.

EXEMPLE 5

Dans une ampoule de 25 cm3, on introduit successivement :

- $RuCl_3$, $3H_2O$ $10^{-4}$ mole
- TPPTS 4,3 x $10^{-4}$ mole
- isopropanol 9 g
- eau 4 g
- prénal 20 x $10^{-3}$ mole

L'ampoule est introduite dans un autoclave de 125 cm3 placé dans une enceinte permettant une agitation par secousses. On purge à l'hydrogène. On fixe la température à 36°C et la pression à 20 bars.

Après 3 heures 30 minutes, l'analyse par chromatographie en phase gazeuse montre que :

– le taux de transformation du prénal est de 89 %

– la répartition des produits d'hydrogénation est la suivante :

. prénol : 88 %
. alcool isoamylique : 1 %

La sélectivité en prénol est de 98 %.

EXEMPLE 6

Dans une ampoule de verre de 25 cm3, on introduit successivement :

- $RuCl_3$, $3H_2O$ 1,22 x $10^{-4}$ mole
- triphénylphosphine 4,7 x $10^{-4}$ mole
- toluène 7 cm3

- éthanol 3 cm3
- citral $9,4 \times 10^{-3}$ mole

L'ampoule est introduite dans un autoclave de 125 cm3 placé dans une enceinte permettant une agitation par secousses. On purge 3 fois avec de l'hydrogène puis on fixe la température à 35°C et la pression d'hydrogène à 50 bars.

Après 7 heures, l'analyse par chromatographie en phase gazeuse montre que :

– le taux de transformation du citral est de 96 %

– la répartition des produits d'hydrogénation est la suivante :

. nérol/géraniol : 96 %
. citronellol : 1,3 %
. citronellal : non détectable
. tétrahydrogéraniol : 0,4 %

La sélectivité en nérol/géraniol est de 97 %.

## Revendications

1. Procédé de préparation d'alcools insaturés de formule générale :

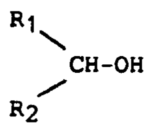

dans laquelle $R_1$ et $R_2$, identiques ou différents, représentent un atome d'hydrogène ou un radical aliphatique saturé ou non saturé, éventuellement substitué par un radical alicyclique saturé ou non saturé ou par un radical aromatique, ou un radical alicyclique saturé ou non saturé ou un radical aromatique, ou bien $R_1$ et $R_2$ forment ensemble un radical alicyclique non saturé, étant entendu que :

– $R_1$ et $R_2$ ne peuvent pas représenter simultanément un atome d'hydrogène,

– l'un au moins des radicaux $R_1$ ou $R_2$ contient une double liaison éthylénique,

– les radicaux aliphatiques, alicycliques ou aromatiques peuvent être éventuellement substitués par un ou plusieurs radicaux, identiques ou différents, choisis parmi les radicaux alcoyles contenant 1 à 4 atomes de carbone, hydroxy ou alcoxy dont la partie alcoyle contient 1 à 4 atomes de carbone, par hydrogénation sélective au moyen d'hydrogène d'un composé carbonylé de formule générale :

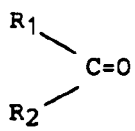

dans laquelle $R_1$ et $R_2$ sont définis comme ci-dessus, caractérisé en ce que l'on opère en milieu liquide homogène en présence d'un catalyseur à base de ruthénium choisi parmi les hydrures et les dérivés halogénés du ruthénium complexés par un ligand ou associés à un ligand éventuellement générés in situ.

2. Procédé selon la revendication 1 caractérisé en ce que le catalyseur est choisi parmi $RuH_2\ (PPh_3)_4$, $RuOAc\ (PPh_3)_4$, $RuCl_2\ (PPh_3)_4$ et les associations d'un sel ou d'un oxyde de ruthénium avec un ligand choisi parmi les phosphines.

3. Procédé selon la revendication 2 caractérisé en ce que le catalyseur est constitué par l'association d'un halogénure de ruthénium avec un ligand choisi parmi les phosphines.

4. Procédé selon la revendication 3 caractérisé en ce que l'halogénure de ruthénium est le chlorure de ruthénium $RuCl_3$, $3H_2O$ et la phosphine est choisie parmi le triphénylphosphine et la tri(métasulfophényl) phosphine.

5. Pocédé selon l'une des revendications 1 à 4 caractérisé en ce que l'on opère dans un solvant organique polaire choisi parmi les alcools éventuellement associé à un solvant organique non polaire choisi parmi les hydrocarbures aliphatiques, cycloaliphatiques ou aromatiques, les éthers et les esters.

6. Procédé selon la revendication 5 caractérisé en ce que le solvant ou le mélange de solvants contient une quantité d'eau qui peut atteindre 35 % du volume total.

7. Procédé selon l'une des revendications 1 à 6 caractérisé en ce que l'on opère sous une pression comprise entre 1 et 200 bars.

8. Procédé selon l'une des revendications 1 à 7 caractérisé en ce que l'on opère à une température comprise entre 0 et 100°C.

9. Procédé selon l'une des revendications 1 à 8 caractérisé en ce que l'on hydrogène sélectivement le prénal en prénol, le citral en géraniol/nérol.

**Claims**

1. Process for the preparation of unsaturated alcohols of the general formula:

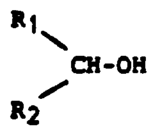

in which $R^1$ and $R^2$, which are identical or different, represent a hydrogen atom, a saturated or unsaturated aliphatic radical optionally substituted by a saturated or unsaturated alicyclic radical or by an aromatic radical, a saturated or unsaturated alicyclic radical or an aromatic radical, or alternatively $R^1$ and $R^2$ together form an unsaturated alicyclic radical, it being understood that:

- $R^1$ and $R^2$ cannot simultaneously represent a hydrogen atom,
- at least one of the radicals $R^1$ and $R^2$ contains an ethylenic double bond, and
- the aliphatic, alicyclic or aromatic radicals can optionally be substituted by one or more identical or different radicals selected from alkyl radicals containing 1 to 4 carbon atoms, hydroxyl radicals and alkoxy radicals of which the alkyl part contains 1 to 4 carbon atoms, by selective hydrogenation, by means of hydrogen, of a carbonyl compound of the general formula:

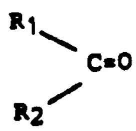

in which $R^1$ and $R^2$ are as defined above, characterised in that the operation is carried out in a homogeneous liquid medium and in the presence of a ruthenium-based catalyst selected from hydrides and halogen derivatives of ruthenium complexed by a ligand or associated with a ligand, which may be generated in situ.

2. Process according to claim 1, characterised in that the catalyst is selected from $RuH_2(PPh_3)_4$, $Ru(OAc)(PPh_3)_4$, $RuCl_2(PPh_3)_4$ and associations of a salt or an oxide of ruthenium with a ligand selected from phosphines.

3. Process according to claim 2, characterised in that the catalyst consists of the association of a ruthenium halide with a ligand selected from phosphines.

4. Process according to claim 3, characterised in that the ruthenium halide is ruthenium chloride, $RuCl_3.3H_2O$, and the phosphine is selected from triphenylphosphine and tri(meta-sulphophenyl)phosphine.

5. Process according to one of claims 1 to 4, characterised in that the reaction is carried out in a polar organic solvent selected from alcohols, optionally in combination with a nonpolar organic solvent selected from aliphatic, cycloaliphatic and aromatic hydrocarbons, ethers and esters.

6. Process according to claim 5, characterised in that the solvent or solvent mixture contains an amount of water which can be as much as 35% of the total volume.

7. Process according to one of claims 1 to 6, characterised in that the reaction is carried out under a pressure of between 1 and 200 bars.

8. Process according to one of claims 1 to 7, characterised in that the reaction is carried out at a temperature of between 0 and 100°C.

9. Process according to one of claims 1 to 8, characterised in that prenal is hydrogenated selectively to prenol, citral to geraniol/nerol.

## Patentansprüche

1. Verfahren zur Herstellung ungesättigter Alkohole der allgemeinen Formel:

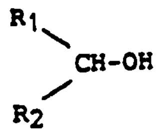

in welcher $R_1$ und $R_2$ unabhängig voneinander ein Wasserstoffatom oder einen gesättigten oder ungesättigten aliphatischen Rest, gegebenenfalls substituiert durch einen gesättigten oder ungesättigten alicyclischen Rest oder durch einen aromatischen Rest, oder einen gesättigten oder ungesättigten alicyclischen Rest oder einen aromatischen Rest darstellen oder $R_1$ und $R_2$ zusammen einen ungesättigten alicyclischen Rest bilden, wobei selbstverständlich:

- $R_1$ und $R_2$ nicht gleichzeitig ein Wasserstoffatom darstellen können,
- zumindest einer der Reste $R_1$ oder $R_2$ eine äthylenische Doppelbindung enthält,
- die aliphatischen, alicyclischen oder aromatischen Reste gegebenenfalls durch einen oder mehrere gleiche oder verschiedene Reste, ausgewählt aus den Alkylresten mit 1 bis 4 Kohlenstoffatomen, den Hydroxyresten oder Alkoxyresten, deren Alkylteil 1 bis 4 Kohlenstoffatome enthält, substituiert sein können, durch selektive Hydrierung einer Carbonylverbindung der allgemeinen Formel:

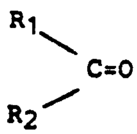

in welcher $R_1$ und $R_2$ die obige Bedeutung haben, mit Wasserstoff, dadurch gekennzeichnet, daß man in homogenem, flüssigem Milieu in Gegenwart eines Katalysators auf Rutheniumbasis, ausgewählt aus den Hydriden und Halogenderivaten von Ruthenium im Komplex mit einem Liganden oder assoziiert mit einem Liganden, die gegebenenfalls in situ erzeugt werden, arbeitet.

2. Verfahren nach Anspruch 1, dadurch gekennzeichnet, daß der Katalysator ausgewählt ist aus $RuH_2(PPh_3)_4$, $RuOAc(PPh_3)_4$, $RuCl_2(PPh_3)_4$, und den Kombinationen eines Rutheniumsalzes oder -oxids mit einem Liganden, ausgewählt aus den Phosphinen.

3. Verfahren nach Anspruch 2, dadurch gekennzeichnet, daß der Katalysator aus der Kombination eines Rutheniumhalogenids mit einem Liganden, ausgewählt aus den Phosphinen, besteht.

4. Verfahren nach Anspruch 3, dadurch gekennzeichnet, daß das Rutheniumhalogenid Rutheniumchlorid $RuCl_3$, $3H_2O$ ist und das Phosphin ausgewählt ist aus Triphenylphosphin und Tri(metasulfophenyl)phosphin.

5. Verfahren nach einem der Ansprüche 1 bis 4, dadurch gekennzeichnet, daß man in einem polaren organischen Lösungsmittel, ausgewählt aus den Alkoholen, gegebenenfalls in Kombination mit einem nicht polaren organischen Lösungsmittel, ausgewählt aus den aliphatischen, cycloaliphatischen oder aromatischen Kohlenwasserstoffen, den Äthern und den Estern, arbeitet.

6. Verfahren nach Anspruch 5, dadurch gekennzeichnet, daß das Lösungsmittel oder die Lösungsmittelmischung eine Wassermenge enthält, die 35 % des Gesamtvolumens erreichen kann.

7. Verfahren nach einem der Ansprüche 1 bis 6, dadurch gekennzeichnet, daß man unter einem Druck zwischen 1 und 200 bar arbeitet.

8. Verfahren nach einem der Ansprüche 1 bis 7, dadurch gekennzeichnet, daß man bei einer Temperatur zwischen 0 und 100°C arbeitet.

9. Verfahren nach einem der Ansprüche 1 bis 8, dadurch gekennzeichnet, daß man selektiv hydriert: Prenal zu Prenol, Citral zu Geraniol/Nerol.